# EUROPEAN PATENT SPECIFICATION

(11) **EP 1 478 425 B1**
(45) Date of publication and mention of the grant of the patent: **02.04.2008**
(21) Application number: 03710222.5
(22) Date of filing: 17.02.2003
(51) Int. Cl.: A61M 25/00, B32B 1/08, B29C 47/06

(54) **MULTILAYER TUBULAR STRUCTURE WITH APPLICATION IN CATHETER MANUFACTURING**
MEHRLAGIGE TUBULÄRE STRUKTUR MIT ANWENDUNG IN DER HERSTELLUNG VON KATHETERN
STRUCTURE TUBULAIRE A PLUSIEURS COUCHES AVEC APPLICATION DANS LA FABRICATION DE CATHETER

(30) Priority: 26.02.2002 IT BS20020024 U
(43) Date of publication of application: 24.11.2004
(73) Proprietor: Invatec S.r.l, 25030 Roncadelle, Brescia (IT)
(72) Inventor: VENTURELLI, Andrea, I-25030 Roncadelle (IT)
(74) Representative: Crippa, Paolo Ernesto
(86) International application number: PCT/IT2003/000086
(87) International publication number: WO 2003/072177

(56) References cited:
- EP-A- 0 029 185
- EP-A- 0 873 759

## Description

### Field of the Invention

This invention concerns catheters in general and refers in particular to an improved multilayer extruded tubing suitable for producing intravascular or similar catheters.

### Prior Art

A trilayer coextruded tubular body for use in medical tubing and medical devices utilising such tubing has already been proposed. A trilayer tubular body, used in a large number of devices for therapeutic and diagnostic purposes is described for example in U.S.-A-6 165 166.

This tubular body comprises an outer layer, an inner layer and an intermediate bonding layer between the outer and inner layers. The outer layer is made of a polymer, such as polyester, polyamide or a combination of both, having a first glass transition temperature. The inner layer, which forms at least one longitudinal lumen, is made up of a lubricious polymer, such as an olefinic or a fluorinate polymer, having a third glass transition temperature. The intermediate layer is made up of a functionalised polymer, such as olefinic modified with either anhydride or acrylate, having a second glass transition temperature.

The outer layer is to provide the resulting tubing with the characteristics of resistance, pushability , torsion, flexibility and weldability, whereas the inner lubricious layer enables the guide wire or any other means inserted into it to slide freely, and the intermediate layer stably bonds the otherwise incompatible and subject to detaching outer and inner layers.

However, the trilayers each have a uniform thickness along all the length of the tubular body and nothing in the abovementioned U.S. patent suggests that it could be otherwise.

In the catheters, the slippery aspect of the lumen or lumens formed by the inner layer is important, but also important if not even more important still are the mechanical characteristics they must have. It is therefore necessary for the coextruded structure of a tubular body for producing catheters and medical devices to really meet these requirements.

### Object and Summary of the invention

It is in fact the objective of the present invention to make and provide a multilayer tubular body for catheter structures where a greater importance is given to the mechanical characteristics of resistance, torsion, flexibility and pushability through a specific combination of the coextruded layers and a different configuration of the outer layer.

This objective is achieved with a trilayer tubular body in which the outer layer has on the inside, in cross section, sectors which penetrate into the intermediate layer, in this way reducing the presence of the intermediate layer and locally increasing the thickness of the outer layer in order to increase the mechanical properties of the resulting tubular body.

### Brief Description of the Drawings

Greater detail of the invention will become clear from the continuation of the present specification made with reference to the enclosed indicative and non-limiting drawings, in which:
Fig. 1 is an enlarged cross-section view of a trilayer tubular body; and
Fig. 2 is a view in perspective of a length of tubular body.

### Detailed Description of the Invention

As shown, the tubular body includes an outer layer 11, an inner layer 12 and an intermediate layer 13. Said tubular body can be extruded, extruding the three layers together and choosing an appropriate polymer for each one. Each layer has a given thickness and the inner layer forms at least one longitudinal lumen 14.

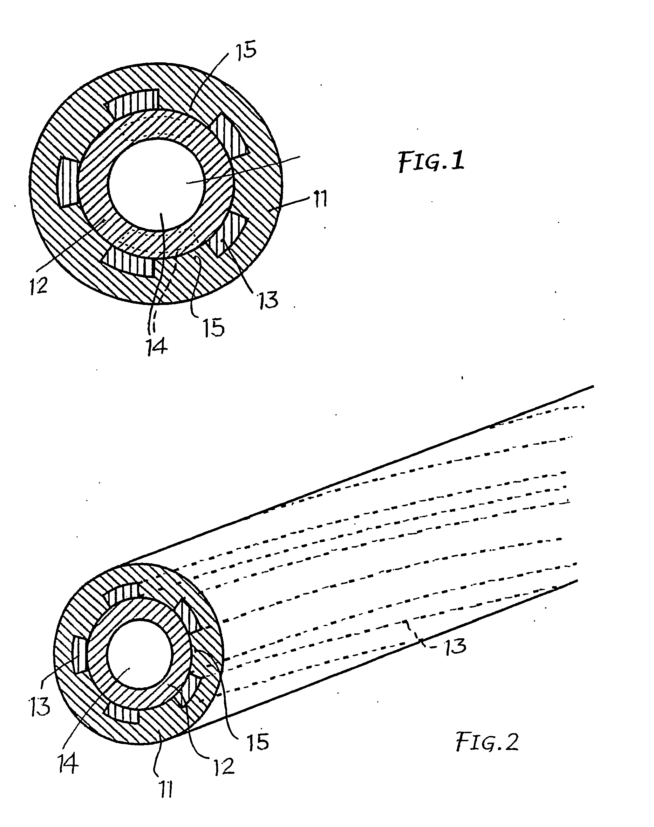

In particular, the outer layer has on the inside, in cross section - Fig. 1, protuberances or sectors 15, of the same or different in size, which penetrate into the thickness of the intermediate layer 13 which in this way is discontinuous and has the relevant joining sectors which alternate with the internal sectors 15 of said outer layer. The internal sectors 15 of the outer layer and in the same way the sectors formed by the intermediate layer 13 may be provided running lengthwise along the tubular body parallel to the axis of the latter or running helicoidally as shown in Fig. 2.

The outer layer 11 is committed to establishing the mechanical properties of the whole plus its weldability with other components of a catheter. The inner layer 12 ensures the smooth movement of the elements which are inserted into it. The presence of the intermediate layer 13, usually having mechanical properties inferior to those of the outer layer 11, according to this invention is limited, allowing an increase in the ratio and contribution of the outer layer 11 without jeopardizing, even if discontinuous and reduced to sectors, the bond between the outer and inner layers to avoid them detaching.

The result is, as said above, particular attention being paid to the mechanical characteristics of the tubular body and consequently to the catheter device to be made, without influencing the normal sizes and other functional aspects of the body itself.

## Claims

1. A multilayer tubular body in particular for the manufacture of catheter devices, comprising an outer layer (11) made of a polymer with the characteristics of resistance, pushability, torsion, flexibility and weldability, an inner layer (12) made of a polymer with lubricious characteristics and forming at least one longitudinal lumen, and an intermediate bonding layer (13) between the outer and inner layers, **characterised by** the fact that the intermediate bonding layer (13) is, in cross-section, in the form of circular sectors having spaces in between, and the outer layer (11) has on the inside parts which alternate with the sectors of the intermediate layer (13).

2. Multilayer tubular body according to claim 1, wherein the outer layer (11) has internal protuberances (15) extending longitudinally, the intermediate layer (13) is discontinuous having spaced apart sectors, and the internal protuberances (15) of outer layer (11) are inserted into the gaps in the intermediate layer sectors.

3. Multilayer tubular body according to claims 1 and 2, wherein the outer layer (11) has internal protuberances (15) which alternate in turns with the gaps in the intermediate layer sectors (13), said internal protuberances and the intermediate layer sectors having the same thickness.

4. Multilayer tubular body according to the previous claims, wherein the internal protuberances (15) of the outer layer (11) and the sectors forming the intermediate bonding layer (13) run lengthways either parallel to or helicoidally to the axial of the body.

## Patentansprüche

1. Mehrschichtiger Schlauchkörper, insbesondere für die Herstellung von Katheter vorrichtungen, umfassend eine Außenschicht (11 gefertigt aus einem Polymer mit den Eigenschaften Festigkeit, Schiebbarkeit, Verdrehbarkeit, Flexibilität und Schweißbarkeit,
eine Innenschicht (12) aus einem Polymer mit Schmiereigenschaften und wenigstens ein längliches Lumen bildend und
eine Zwischen-Bondingschicht (13) zwischen der Außen- und der Innenschicht,
**gekennzeichnet dadurch, dass** die Zwischen-Bondingschicht (13) im Querschnitt in der Form von kreisförmigen Sektoren ist, die Abstände dazwischen aufweisen, und die Außenschicht (11) auf der Innenseite Teile aufweist, die mit den Sektoren der Zwischenschicht (13) alternieren.

2. Mehrschichtiger Schlauchkörper nach Anspruch 1, wobei die Außenschicht (11) innere Ausstülpungen (15) aufweist, die sich in Längsrichtung erstrecken, die Zwischenschicht (13) unterbrochen mit beabstandeten Sektoren ist und die inneren Ausstülpungen (15) der Außenschicht (11) in die Aussparungen in den Zwischenschichtsektoren eingefügt sind.

3. Mehrschichtiger Schlauchkörper nach Anspruch 1 und 2, wobei die Außenschicht (11) interne Ausstülpungen (15) aufweist, die im Wechsel mit den Aussparungen in den Zwischenschichtsektoren (13) alternieren, wobei die inneren Ausstülpungen und die Zwischenschichtsektoren dieselbe Dicke haben.

4. Mehrschichtiger Schlauchkörper nach einem der vorhergehenden Ansprüche, wobei die inneren Ausstülpungen (15) der Außenschicht (11) und die Sektoren, die die Zwischen-Bondingschicht (13) bilden, längs entweder parallel mit oder in Schraubenlinie zu der Achse des Körpers verlauFen.

## Revendications

1. Corps tubulaire à couches multiples, en particulier pour la fabrication de dispositifs de cathéters, comprenant une couche externe (11) faite d'un polymère aux caractéristiques de résistance, aptitude à être poussé, torsion, flexibilité et soudabilité, une couche interne (12) faite d'un polymère aux caractéristiques de lubricité et formant au moins une lumière longitudinale, et une couche de liaison intermédiaire (13) entre les couches externe et interne, **caractérisé en ce que** la couche de liaison intermédiaire (13) est, en section transversale, en forme de secteurs circulaires ayant des espaces entre eux, et la couche externe (11) a ses parties internes qui alternent avec les secteurs de la couche intermédiaire (13).

2. Corps tubulaire à couches multiples selon la revendication 1, dans lequel la couche externe (11) a des protubérances internes (15) s'étendant longitudinalement, la couche intermédiaire (13) est discontinue, ayant des secteurs espacés, et les protubérances internes (15) de la couche externe (11) sont insérées dans les interstices dans les secteurs de couche intermédiaire.

3. Corps tubulaire à couches multiples selon les revendications 1 et 2, dans lequel la couche externe (11) a des protubérances internes (15) qui alternent en spires avec les interstices dans les secteurs de couche intermédiaire (13), lesdites protubérances internes et les secteurs de couche intermédiaire ayant la même épaisseur.

4. Corps tubulaire à couches multiples selon les revendications précédentes, dans lequel les protubérances internes (15) de la couche externe (11) et les secteurs formant la couche de liaison intermédiaire (13) s'étendent dans le sens de la longueur, soit parallèlement, soit hélicoïdalement par rapport à l'axe du corps.
